# EUROPEAN PATENT SPECIFICATION

(11) **EP 4 227 083 B1**
(45) Date of publication and mention of the grant of the patent: **13.08.2025**
(21) Application number: 21877429.7
(22) Date of filing: 29.09.2021
(51) Int. Cl.: B32B 25/10, B32B 7/025

(54) **ELECTROCONDUCTIVE RUBBERIZED FABRIC**
ELEKTRISCH LEITFÄHIGES GUMMIERTES GEWEBE
TISSU CAOUTCHOUTÉ CONDUCTEUR

(30) Priority: 07.10.2020 JP 2020169662
(43) Date of publication of application: 16.08.2023
(73) Proprietor: NOK Corporation, Tokyo 105-8585 (JP)
(72) Inventor: HAYASHI, Taisei, Tsujido-Shinmachi, Fujisawa-shi, Kanagawa 251- 0042 (JP); KUBO, Masayuki, Tsujido-Shinmachi, Fujisawa-shi, Kanagawa 251- 0042 (JP); HAYASHI, Takahiro, Tsujido-Shinmachi, Fujisawa-shi, Kanagawa 251- 0042 (JP)
(74) Representative: Global IP Europe Patentanwaltskanzlei
(86) International application number: PCT/JP2021/035751
(87) International publication number: WO 2022/075128

(56) References cited:
- WO-A1-2005/118281
- CN-A- 110 421 933
- JP-A- H01 152 695
- JP-B2- H07 120 868
- JP-Y2- H0 346 903

## Description

### FIELD

The present invention relates to an electroconductive rubberized fabric. More specifically, the present invention relates to an electroconductive rubberized fabric that can be used as a bioelectrode capable of stably detecting a weak biosignal.

### BACKGROUND

Conventionally, medical devices such as an electrocardiogram, an electroencephalograph, or an electromyograph have been used in medical fields. Such a medical device captures, as a biosignal, fluctuation in an electric potential generated from a human body, and displays the captured biosignal. Thereby, a health condition or the like can be recognized. Wearable information devices such as an active tracker (activity amount meter) have widely spread, in recent years, among people who take care of health. The wearable information device is attached to an arm, a wrist, or the like, and can collect various daily biological information by measuring a body temperature, a heart rate, a blood pressure, or the like over time, measuring an activity amount such as a walking distance, the number of steps, or the like, or measuring sleep duration, or a depth or quality of sleep, or the like, for example.

Another technique whose development is being widely advanced is that in which a biosignal consequent on operation or movement of a driver is detected to operate and control a car navigation system or any of various on-vehicle devices mounted on an automobile. Thereby, the biosignal can be reflected in safe driving or the like. For example, an accident is prevented, or a driver is notified of danger in advance.

Thus, many developments for various electronic devices such as wearable information devices and on-vehicle devices are underway concerning detection and measurement techniques of sensors and the like for highly accurately detecting a biosignal generated by a human body. Particularly, performance improvement of a bioelectrode attached directly to a human body is expected for highly accurately detecting a change in a weak biosignal.

The bioelectrode is attached to a part of a body surface such as skin of a human body, for example. The bioelectrode captures a change in an amount of electric current or the like as a biosignal flowing in the body surface or an inside of the human body. In this case, for example, electroconductive rubber is often used in order to detect a weak biosignal.

However, the electroconductive rubber itself has a disadvantage such as low mechanical strength. For this reason, a problem such as easy tearing or breaking of the electroconductive rubber can occur. The problem is caused by excessive external force applied to the electroconductive rubber at the time of impact application when the electroconductive rubber is used as the bioelectrode or at the time of manufacturing the bioelectrode (e.g., at the time of sewing). Thus, when the electroconductive rubber is used as the bioelectrode, improvement in durability of the bioelectrode is required.

For example, the electroconductive rubber and woven cloth (fabric) are stuck integrally to each other, using a well-known forming-processing technique such as calender forming. This results in formation of an electroconductive rubberized fabric (hereinafter, referred to as "two-layer structure electroconductive rubberized fabric") having a two-layer structure in which the electroconductive rubber and the woven cloth are superimposed on each other. Thereby, a biosignal can be satisfactorily detected by the electroconductive rubber, and mechanical strength can be improved by the woven cloth. Thus, the two-layer structure electroconductive rubberized fabric having superior mechanical strength can be adopted as the bioelectrode.

An electroconductive rubberized woven cloth known as another example of the bioelectrode is formed by impregnating an insulating fabric with an electroconductive material and then providing a wiring, an electrode, and the like (refer to Japanese Patent Application Laid-open Publication No. 2014-151018). In addition, a known stretchable electrode and wiring sheet that have stretchability include woven cloth as base materials to which an electrode and a wiring are connected using electroconductive rubber (refer to International Publication No. WO2016/114298 A).

JP H03-046903 Y2 discloses an electroconductive rubberized fabric comprising:
a base fabric layer formed of an insulating base fabric and including a first base fabric layer surface and a second base fabric layer surface;
an electroconductive rubber layer formed by superimposing electroconductive rubber on the first base fabric layer surface; and
an electroconductive paste layer formed by coating the second base fabric layer surface with an electroconductive paste material having an electric resistance value lower than that of the electroconductive rubber.

### BRIEF SUMMARY

### TECHNICAL PROBLEM

When the two-layer structure electroconductive rubberized fabric is used as the bioelectrode, the following problem can occur.

The insulating woven cloth having a property of preventing electricity from passing therethrough and the electroconductive rubber having a property of allowing electricity to passing therethrough are stuck to each other. Thereby, the two-layer structure electroconductive rubberized fabric is integrally formed. For this reason, the insulating woven cloth can cause an overall electric resistance value of the two-layer structure electroconductive rubberized fabric to become larger than that when the electroconductive rubber is used alone. As a result, detection accuracy of a biosignal can be lowered, and a change in a weak biosignal cannot be detected. In other words, there is a possibility that sufficient performance for the bioelectrode cannot be exhibited.

Specifically, the bioelectrode formed of the two-layer structure electroconductive rubberized fabric is used in a part of a seat, an armrest, or a steering wheel of an automobile, for operation and control of an on-vehicle device installed in a vehicle interior of the automobile. Thereby, a bioinformation (biosignal) of a driver is acquired. In this case, the bioelectrode itself is generally formed in an elongated shape in many cases.

The bioelectrode itself is electrically connected, via a metal terminal, to a wiring extending from the on-vehicle device or the like. However, there is a possibility that an electric resistance value in the elongated bioelectrode becomes high, due to influence of the insulating woven cloth, at an electrode face in the bioelectrode that is distant from the metal terminal connected to the wiring. Then, it can become difficult for electric current to flow in an amount enough to be captured as a biosignal. Thus, detection accuracy of a change in a weak biosignal at the electrode face can decline.

For example, a conceivable measure to solve such a problem is to increase the number of metal terminals attached to the bioelectrode, thereby preventing an increase in a distance from the metal terminal to the electrode face. However, an increase in the number of the installed metal terminals causes the bioelectrode itself to become bulky, and a mounting position thereof is restricted. In addition, visual quality of the bioelectrode itself deteriorates, and aesthetic appearance of the vehicle interior where the bioelectrode is installed is impaired. Further, an increase in the number of the installed metal terminals causes an increase in cost. Furthermore, for example, when a large number of accessories such as the metal terminals are attached to the steering wheel, steerability of the steering wheel itself can decline.

An object of the present invention is to provide an electroconductive rubberized fabric that can be used as a bioelectrode and that can suppress an increase in an electric resistance value and prevent a decline in detection accuracy of a weak biosignal.

### SOLUTION TO PROBLEM

The above-mentioned problem is solved by an electroconductive rubberized fabric defined in the appended independent claim. Further advantageous effects can be achieved by preferred embodiments defined in the appended dependent claims.

The electroconductive rubberized fabric according to the present invention includes:
a base fabric layer formed of an insulating base fabric and including a first base fabric layer surface and a second base fabric layer surface;
an electroconductive rubber layer formed by superimposing electroconductive rubber on the first base fabric layer surface; and
an electroconductive paste layer formed by coating the second base fabric layer surface with an electroconductive paste material having an electric resistance value lower than that of the electroconductive rubber;
characterized in that the electroconductive paste layer covers only a part of the second base fabric layer surface.

### ADVANTAGEOUS EFFECTS

According to the present invention, it is possible to provide an electroconductive rubberized fabric that can be used as a bioelectrode and that can suppress an increase in an electric resistance value and prevent a decline in detection accuracy of a weak biosignal.

### BRIEF DESCRIPTION OF DRAWINGS

FIG. 1 is an illustration depicting a schematic configuration of the electroconductive rubberized fabric according to a first embodiment.
FIG. 2 is a cross-sectional view taken along the line A-A' in FIG. 1.
FIG. 3 is an illustration depicting a schematic configuration of an electroconductive rubberized fabric according to a first reference example not forming part of the present invention.
FIG. 4 is a cross-sectional view taken along the line B-B' in FIG. 3.
FIG. 5 is an illustration depicting a schematic configuration for a method of measuring an electric resistance value of the electroconductive rubberized fabric.
FIG. 6 is an illustration depicting one example of a wiring method at the time of measuring an electric resistance value of the electroconductive rubberized fabric.

### DETAILED DESCRIPTION

The following describes embodiments of an electroconductive rubberized fabric according to the present invention in detail with reference to the drawings. The electroconductive rubberized fabrics according to the embodiments are not limited to the below-described ones. Various design changes, modifications, improvements, and the like can be made without departing from the scope of the present invention defined by the appended independent claim.

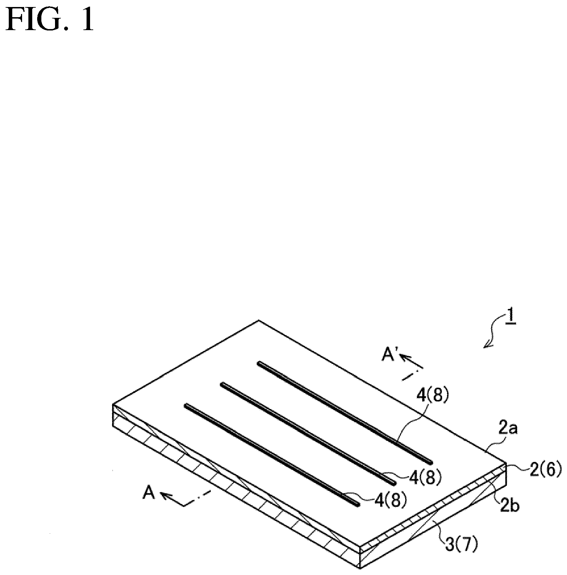

The electroconductive rubberized fabrics 1 and 5 according to the present embodiment each have a three-layer structure in which three layers are superimposed on each other as illustrated in FIG. 1 to FIG. 4. The electroconductive rubberized fabrics 1 and 5 each include a base fabric layer 2, an electroconductive rubber layer 3, and an electroconductive paste layer 4 or 9.

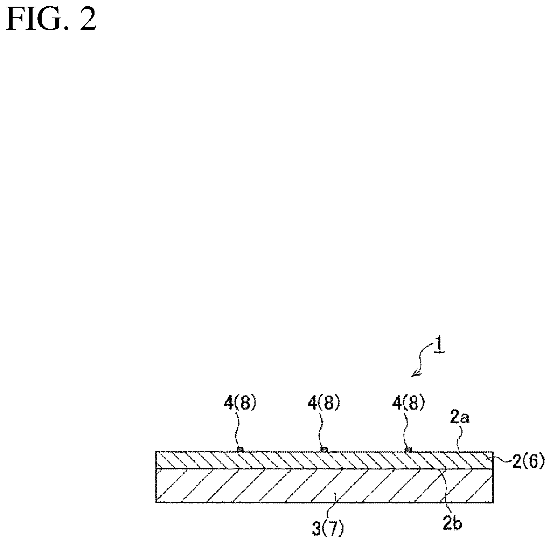

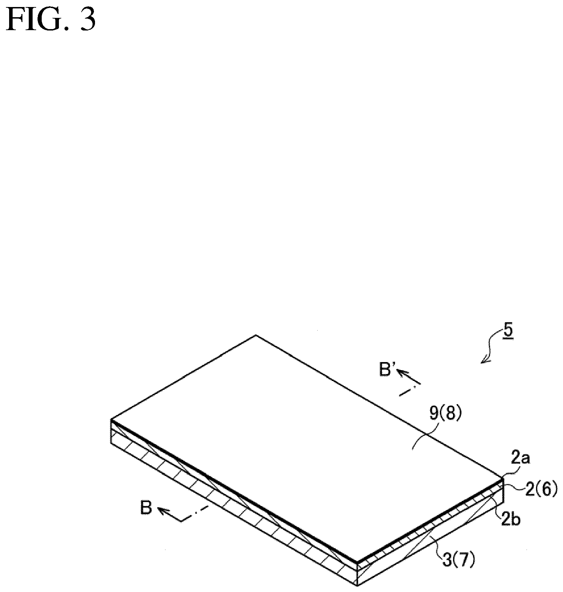

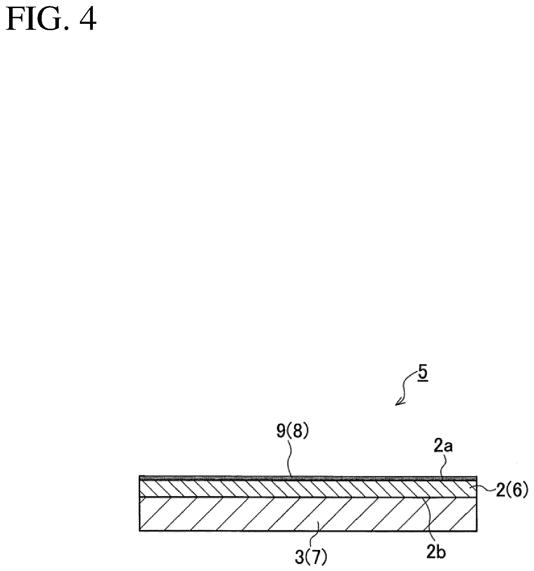

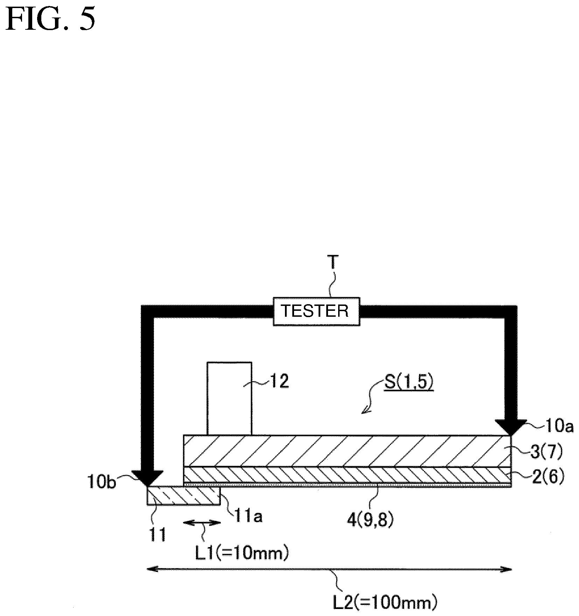

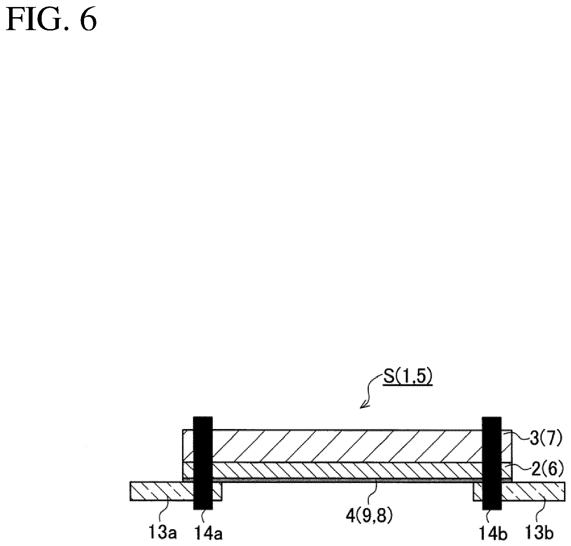

FIG. 1 is an illustration depicting a schematic configuration of the electroconductive rubberized fabric 1 according to the first embodiment. FIG. 2 is a cross-sectional view taken along the line A-A' in FIG. 1. FIG. 3 is an illustration depicting a schematic configuration of the electroconductive rubberized fabric 5 according to the first reference example. FIG. 4 is a cross-sectional view taken along the line B-B' in FIG. 3. FIG. 5 is an illustration depicting a schematic configuration for a method of measuring an electric resistance value of the electroconductive rubberized fabric 1 or 5. FIG. 6 is an illustration depicting one example of a wiring method at the time of measuring an electric resistance value of the electroconductive rubberized fabric 1 or 5.

### First Embodiment

The electroconductive rubberized fabric 1 according to the first embodiment includes the base fabric layer 2, the electroconductive rubber layer 3, and the electroconductive paste layer 4, as illustrated in FIG. 1 and FIG. 2. The base fabric layer 2 is formed of a base fabric 6. The base fabric 6 is made of an insulating material. The electroconductive rubber layer 3 is formed of electroconductive rubber 7. The electroconductive rubber 7 contacts against the entirety of a first base fabric layer surface 2b of the base fabric layer 2. The electroconductive paste layer 4 is formed by coating an electroconductive paste material 8 on a part of a second base fabric layer surface 2a of the base fabric layer 2.

The electroconductive rubberized fabric 1 has a three-layer structure in which the base fabric layer 2 is sandwiched between the electroconductive rubber layer 3 and the electroconductive paste layer 4. The electroconductive paste layer 4 is formed so as to cover only a part of the second base fabric layer surface 2a.

The electroconductive rubber layer 3 is the electroconductive rubber 7 that has been formed in a sheet shape having a predetermined thickness, as illustrated in FIG. 1 and FIG. 2. The electroconductive rubber 7 as an unvulcanized rubber material is, for example, heated and pressed between a plurality of rolls while being extruded onto the first base fabric layer surface 2b. Thus, the base fabric layer 2 and the electroconductive rubber layer 3 are formed integrally with each other by, for example, a well-known calender forming of sticking the electroconductive rubber layer 3 to the base fabric layer 2 while forming the electroconductive rubber layer 3. A two-layer structure constituted of the base fabric layer 2 and the electroconductive rubber layer 3 corresponds to a two-layer structure electroconductive rubberized fabric.

The electroconductive rubberized fabric 1 includes the electroconductive paste layer 4 as well as a configuration of the two-layer structure electroconductive rubberized fabric constituted of the base fabric layer 2 and the electroconductive rubber layer 3. The electroconductive paste layer 4 is provided by coating the electroconductive paste material 8 on the second base fabric layer surface 2a that is a surface opposite to the first base fabric layer surface 2b. Thereby, the electroconductive rubberized fabric 1 having a three-layer structure as a whole is completed.

A method for manufacturing the electroconductive rubberized fabric 1 is not particularly limited. For example, the two-layer structure electroconductive rubberized fabric may be manufactured in advance, and then, the electroconductive paste layer 4 may be formed by coating the electroconductive paste material 8 on the second base fabric layer surface 2a, using well-known coat forming means such as dipping, screen printing, or a squeegee. Alternatively, the electroconductive paste layer 4 may be formed on the second base fabric layer surface 2a, and then, the base fabric layer 2 and the electroconductive rubber layer 3 may be formed integrally with each other.

The mutual sticking of the base fabric layer 2 and the electroconductive rubber layer 3 is also not limited to that in which the electroconductive rubber layer 3 is stuck to the base fabric layer 2 while being formed. For example, the electroconductive rubber layer 3 formed in a sheet shape in advance may be stuck to the base fabric layer 2, using an electroconductive adhesive, by a well-known bonding technique or the like. In this case, the adhesive is preferably applied to an entire surface between the base fabric layer 2 and the electroconductive rubber layer 3. In addition, a forming-processing technique other than the calender forming may be used.

A size of the electroconductive rubberized fabric 1 is not particularly limited. In the case of assumed use as a bioelectrode or the like, the entirety of the three layers including the base fabric layer 2, the electroconductive rubber layer 3, and the electroconductive paste layer 4 has, for example, a thickness approximately in a range from 0.1 mm to 10 mm, more preferably a thickness approximately in a range from 0.3 mm to 1 mm. The electroconductive rubberized fabric 1 formed as an elongated body has a length equal to or longer than 50 mm.

The electroconductive paste layer 4 in the first embodiment has a layer thickness equal to or smaller than 1/10 of the sum of layer thicknesses of the base fabric layer 2 and the electroconductive rubber layer 3, as illustrated in FIG. 1 and FIG. 2.

Such a size of the electroconductive rubberized fabric 1 enables suppression of an increase in an electric resistance value, and enables accurate detection of a biosignal, even for an electrode face separated from a metal terminal.

Here, the base fabric 6 used for the base fabric layer 2 is not particularly limited, and various insulating materials can be used for it. For example, a nonwoven fabric made of an aramid fiber mainly used as a base fabric layer of a conventional two-layer structure electroconductive rubberized fabric can be used for it. The nonwoven fabric made of the aramid fiber has a porous structure in which a plurality of voids (not illustrated) are formed inside the base fabric 6.

For this reason, when by a forming-processing technique such as calender forming, the electroconductive rubber 7 is stuck to the base fabric 6 while being pressure-bonded to the base fabric 6 so as to form the electroconductive rubber layer 3, a part of the stuck electroconductive rubber 7 enters the voids of the base fabric 6. When the electroconductive paste material 8 in a paste form having a predetermined viscosity is coated on the second base fabric layer surface 2a, a part of the electroconductive paste material 8 permeates into an inside from the second base fabric layer surface 2a. As a result, at least respective parts of the electroconductive rubber 7 and the electroconductive paste material 8 are mixed with each other in the voids inside the base fabric 6.

Thereby, the electroconductive rubber layer 3 formed on a side of the first base fabric layer surface 2b and the electroconductive paste layer 4 formed on a side of the second base fabric layer surface 2a are electrically connected to each other. Thus, an increase in an electric resistance value of the electroconductive rubberized fabric 1 is suppressed. Thereby, satisfactory electroconductivity is maintained, and the problem at the time of detecting a biosignal is prevented.

The electroconductive rubber 7 is that conventionally used in a well-known electroconductive rubberized fabric. Examples of the electroconductive rubber 7 include electroconductive silicone rubber whose base material is silicone rubber, and electroconductive urethane rubber whose base material is urethane rubber.

The electroconductive rubber 7 may be formed as follows. Electroconductive carbon particles of carbon black, graphite, or the like, and silver paste, silver powder, or the like are appropriately mixed with a base material such as silicone rubber, and are kneaded. Then, the mixture is forming-processed so as to have a desired size, using a predetermined rubber forming technique (a direct pressure forming technique, a direct pressure injection technique, an injection forming technique, or the like). Thereby, the sheet-shaped electroconductive rubber 7 imparted with electroconductivity is formed.

A mixed ratio of the electroconductive carbon particles and the like to the base such as silicone rubber or urethane rubber in the mixture that can be used here is not particularly limited. The mixed ratio is in a range from 10% by weight to 70% by weight, for example, and is more preferably in a range from 20% by weight to 50% by weight.

Similarly to the electroconductive rubber 7, the one usable as the electroconductive paste material 8 is a mixture in which 50 to 500 parts by weight for example, more preferably 100 to 300 parts by weight of electroconductive carbon particles and the like are mixed with 100 parts by weight of a base material (silicone rubber or the like) as a base. However, viscosity and the like thereof is adjusted as compared with the electroconductive rubber 7, in order to enable the coating to the second base fabric layer surface 2a.

A method for the coating to the base fabric layer 2 is not particularly limited. For example, the method may be the coating onto the second base fabric layer surface 2a by a well-known dispenser, or may be the one that uses a printing technique such as screen printing. Viscosity and the like of the electroconductive paste material 8 is adjusted depending on each of the coating methods.

The electroconductive rubberized fabric 1 according to the first embodiment includes the electroconductive paste layer 4 that is provided on the second base fabric layer surface 2a. Thereby, an increase in an electric resistance value can be suppressed as compared with the conventional two-layer structure electroconductive rubberized fabric. Particularly, the base fabric layer 2 has a porous structure, and thus, respective parts of the electroconductive rubber 7 and the electroconductive paste material 8 are mixed with each other in the voids of the porous structure. Thereby, electrical connection between the electroconductive rubber layer 3 and the electroconductive paste layer 4 is secured, and satisfactory electroconductivity of the entire electroconductive rubberized fabric 1 is secured. For this reason, the electroconductive rubberized fabric 1 can be favorably used as a bioelectrode.

The electroconductive paste material 8 forming the electroconductive paste layer 4 has an electric resistance value lower than an electric resistance value of the electroconductive rubber 7. Thereby, in the case of use as a bioelectrode, electric current can easily flow on a side of the electroconductive paste layer 4 used as an electrode face. Thus, the electroconductive rubberized fabric 1 can be more favorably used as a bioelectrode.

The number of metal terminals connected to a bioelectrode can be minimized. The aesthetic appearance is not impaired. Further, the influence on steerability and the like can be suppressed.

### First Reference Example

The electroconductive rubberized fabric 5 according to the first reference example includes the base fabric layer 2, the electroconductive rubber layer 3, and the electroconductive paste layer 9, as illustrated in FIG. 3 and FIG. 4. The base fabric layer 2 is formed of the base fabric 6. The base fabric 6 is made of an insulating material. The electroconductive rubber layer 3 is formed of the sheet-shaped electroconductive rubber 7. The electroconductive rubber 7 contacts against the entirety of the first base fabric layer surface 2b of the base fabric layer 2. The electroconductive paste layer 9 is formed by coating the electroconductive paste material 8 on the entirety of the second base fabric layer surface 2a of the base fabric layer 2. Here, the constituents that are included in the electroconductive rubberized fabric 5 according to the first reference example and that are the same as those in the electroconductive rubberized fabric 1 according to the first embodiment are denoted by the same reference signs, and detailed description thereof is omitted.

The electroconductive rubberized fabric 5 according to the first reference example differs from the electroconductive rubberized fabric 1 according to the first embodiment in that the electroconductive paste layer 9 is formed on the entirety of the second base fabric layer surface 2a. The other constituents of the electroconductive rubberized fabric 5 according to the first reference example are the same as those of the electroconductive rubberized fabric 1 according to the first embodiment. For this reason, detailed description of the base fabric layer 2 and the electroconductive rubber layer 3 is omitted.

The electroconductive paste material 8 forming the electroconductive paste layer 9 is also the same as that in the first embodiment. Accordingly, detailed description of the electroconductive paste layer 9 is also omitted.

However, the electroconductive paste layer 9 needs to be formed on the entirety of the second base fabric layer surface 2a, and thus, the electroconductive paste layer 9 having a uniform thickness can be formed on the second base fabric layer surface 2a by using a squeegee, screen printing, or the like rather than coating by dipping.

The electroconductive rubberized fabric 5 includes the electroconductive paste layer 9 that is formed on the entirety of the second base fabric layer surface 2a, as illustrated in FIG. 3 and FIG. 4. For this reason, detection accuracy of a biosignal on the electrode face is more satisfactory than that in the electroconductive rubberized fabric 1 according to the first embodiment. Thus, a biosignal can be detected stably with high accuracy, which is favorable.

The electroconductive paste material 8 is applied to the entirety of the second base fabric layer surface 2a, as compared with the electroconductive rubberized fabric 1 according to the first embodiment. Thereby, the electroconductive paste layer 9 is formed. Thus, a used amount of the electroconductive paste material 8 inevitably increases, and manufacturing cost can increase.

For this reason, the electroconductive rubberized fabric may be appropriately selectively used, depending on an attached position, an installation environment, and the like at the time of being used as a bioelectrode. For example, in the case of detecting a biosignal in a wide area, the electroconductive rubberized fabric 1 according to the first embodiment is used, and in the case of detecting a biosignal in a local area, the electroconductive rubberized fabric 5 according to the first reference example having higher detection accuracy is used.

### Examples

The following describes the present invention in more detail, based on the examples. However, the present invention is not limited to these examples. Various modifications and improvements other than the following examples can be adopted based on knowledge of those skilled in the art without departing from the scope of the present invention defined by the appended independent claim.

### (1) Used Members and Used Conditions of Electroconductive Paste Material

Base fabric: aramid fiber
Electroconductive rubber: electroconductive urethane rubber and electroconductive silicone rubber
Electroconductive paste material: silver-powder-dispersed silicone rubber
Vulcanization temperature for electroconductive paste material: 150 °C
Vulcanization time for electroconductive paste material: 30 minutes

Table 1 represents measured results of respective electric resistance values (surface resistance values) of the electroconductive urethane rubber, the electroconductive silicone rubber, and the electroconductive paste material that are the used members. Here, the electric resistance values were each measured in a state where a distance between terminals of a tester for measuring the electric resistance value was set at 90 mm. Three test pieces each constituted of the used member whose size is 48 mm in width and 93 mm in length were prepared for the measurement. The average of measured results of the respective test pieces was calculated as the electric resistance value.

**Table 1**

| Used member | Electric resistance value |
|---|---|
| Electroconductive urethane rubber | 7.5 MΩ |
| Electroconductive silicone rubber | 420 Ω |
| Electroconductive paste material | 1.00 Ω |

It is indicated from the measured results of the electric resistance values in Table 1 that the electric resistance value of the electroconductive paste material used for the electroconductive paste layer is lower than each of the electric resistance values (i.e., the surface resistance values) of the electroconductive rubber (the electroconductive urethane rubber and the electroconductive silicone rubber) used for the electroconductive rubber layer. In other words, the electric resistance value of the electroconductive urethane rubber as the electroconductive rubber is 7.5 MΩ, and the electric resistance value of the electroconductive silicone rubber is 420 Ω, whereas the electric resistance value of the electroconductive paste material is 1.00 Ω significantly lower than these.

### (2) Fabrication of Electroconductive Rubberized Fabrics of Examples 1 to 5 and Comparative Examples 1 and 2

The electroconductive rubberized fabrics of the examples 1 to 5 and the comparative examples 1 and 2 were fabricated, using the above-described used members. First, the base fabric made of the aramid fibers and the electroconductive rubber previously forming-processed into a sheet shape were stuck to each other, and heated and pressed between rolls. Thereby, the elongated two-layer structure electroconductive rubberized fabric conventionally well known was formed. Since the forming method by calender-forming processing is well known, detailed description thereof is omitted here. Here, the thus-obtained two-layer structure electroconductive rubberized fabric that uses the electroconductive urethane rubber as the electroconductive rubber and that is not coated with the following electroconductive paste material is the comparative example 1. The thus-obtained two-layer structure electroconductive rubberized fabric that uses the electroconductive silicone rubber as the electroconductive rubber and that is not coated with the following electroconductive paste material is the comparative example 2. In other words, each of the comparative examples 1 and 2 is a well-known two-layer electroconductive rubberized fabric.

In the example 1, the electroconductive paste material as the above-described used member is formed (not illustrated) on the surface (second base fabric layer surface) in the base fabric and opposite to the electroconductive rubber (electroconductive rubber layer) in the two-layer structure electroconductive rubberized fabric. This two-layer structure electroconductive rubberized fabric includes the base fabric and the electroconductive rubber made of the electroconductive urethane rubber that are stuck to each other. Here, one linear figure was formed, using a dispenser, so as to have a predetermined dip height and a predetermined dip amount, and was dried for predetermined time and subjected to curing of the electroconductive paste material. Thereby, the electroconductive paste layer was formed.

Here, the example in which the number of the electroconductive paste layers each constituted of the linear figure is one is represented as "one electroconductive paste line" in Table 2. Hereinafter, each of the examples is represented as "N electroconductive paste lines", depending on the number of the electroconductive paste layers each constituted of the linear figure. Here, the fabricated electroconductive rubberized fabrics are each an elongated body having a rectangular shape as a whole.

The example 2 is fabricated by the same used members and fabrication method as those in the example 1. In the example 2, the three linear electroconductive paste layers (corresponding to "three electroconductive paste lines") are formed on the second base fabric layer surface. In other words, the example 2 corresponds to the shape of the electroconductive rubberized fabric according to the first embodiment illustrated in FIG. 1 and FIG. 2.

The example 3 is fabricated by the same used members and fabrication method as those in the example 1 and the example 2. In the example 3, the five linear electroconductive paste layers (corresponding to "five electroconductive paste lines" not illustrated) are formed on the second base fabric layer surface.

Thus, the respective examples 1 to 3 correspond to the electroconductive rubberized fabric according to the first embodiment, and the mutual difference lies only in the number of the electroconductive paste layers formed on the second base fabric layer surface.

In the example 4, the electroconductive paste material as the above-described used member is formed on the entirety of the surface (second base fabric layer surface) in the base fabric and opposite to the electroconductive rubber (electroconductive rubber layer) in the two-layer structure electroconductive rubberized fabric. This two-layer structure electroconductive rubberized fabric includes the base fabric and the electroconductive rubber made of the electroconductive urethane rubber that are stuck to each other. Here, the electroconductive paste material was coated, using a squeegee and screen printing, so as to be thin and uniform, and was dried for the predetermined time and subjected to curing of the electroconductive paste material. Thereby, the electroconductive paste layer was formed.

The example 5 is fabricated by the same fabrication method as that in the example 4. In the example 5, the electroconductive rubber layer was changed to that of the electroconductive silicone rubber.

In each of the comparative examples 1 and 2, the electroconductive paste layer is formed using the electroconductive paste material, as described above. In the comparative example 1, the electroconductive urethane rubber was used as the electroconductive rubber layer. In the comparative example 2, the electroconductive silicone rubber was used as the electroconductive rubber layer.

### (3) Method for Measuring Electric Resistance Value

Electric resistance values were measured for the electroconductive rubberized fabrics of the examples 1 to 5 and the comparative examples 1 and 2, by the measurement method schematically illustrated in FIG. 5. Here, the electric resistance values were measured using the tester T (model name: CD772) made by Sanwa Electric Instrument Co., Ltd. and having the maximum rated input of 40 MΩ.

A measurement-target sample S constituted of the elongated-body electroconductive rubberized fabric is fixed to wirings 13a and 13b by electroconductive members 14a and 14b formed of electroconductive materials, in the measurement of an electric resistance value, as illustrated in FIG. 6. Thus, the sample S needs to be electrically connected to the wiring 13a and the like on a side of the electroconductive paste layer 4. For this reason, the method of measuring an electric resistance value is adopted.

First, the tester T for measuring an electric resistance value is arranged, and a first terminal 10a connected to the tester T is connected to one end (on a right side in FIG. 5) of the sample S. Further, a second terminal 10b connected to the tester T is connected to one end (on a left side in FIG. 5) of an aluminum foil 11. Here, the aluminum foil 11 extends along the elongated direction from the second terminal 10b toward the first terminal 10a (from the left side to the right side in FIG. 5).

The aluminum foil 11 is covered with the sample S from above such that the electroconductive paste layer 4 contacts with the aluminum foil 11, in a range of a sample close-contact section L1 having a width of 10 mm from a distal end 11a of the aluminum foil. Thereby, the electroconductive aluminum foil 11 closely contacts with a part of the measurement-target sample S. Further, a weight 12 is placed on the sample S from above in order to maintain the close contact state at the time of the measurement. Thus, the state of the close contact between the sample S and the aluminum foil 11 is fixed.

A distance (terminal-to-terminal distance L2) between the first terminal 10a and the second terminal 10b connected to the tester T is set at 100 mm, as illustrated in FIG. 5.

Such a measurement method for an electric resistance value was used in measuring an electric resistance value by the tester T in a state where each of the samples S (the examples 1 to 5 and the comparative examples 1 and 2) was set at a predetermined position. An electric resistance value was measured three times for the same sample S, and an average value thereof was calculated. The results are represented in Table 2.

**Table 2**

| | Electroconductive rubber layer | Electroconductive paste layer | Electric resistance value |
|---|---|---|---|
| Example 1 | Electroconductive urethane rubber | One electroconductive paste line | 6.23 MΩ |
| Example 2 | Electroconductive urethane rubber | Three electroconductive paste lines | 2.57 MΩ |
| Example 3 | Electroconductive urethane rubber | Five electroconductive paste lines | 2.59 MΩ |
| Example 4 | Electroconductive urethane rubber | Entire-surface coating | 1.62 MΩ |
| Example 5 | Electroconductive silicone rubber | Entire-surface coating | 50 to 70 Ω |
| Comparative Example 1 | Electroconductive urethane rubber | None | 40 MΩ or larger (unmeasurable) |
| Comparative Example 2 | Electroconductive silicone rubber | None | 70 to 80 kΩ |

### (4) Conclusion of Measurement Results

As indicated in the measurement results of an electric resistance value in Table 2, the following is indicated. When the electroconductive urethane rubber is used as the electroconductive rubber, the electric resistance value in each of the examples 1 to 4 in which the electroconductive paste layer is provided is significantly lower than the electric resistance value in the comparative example 1 in which the electroconductive paste layer is not provided.

Further, the following is indicated. The electric resistance values in the example 2 and the example 3 in which the three or five linear electroconductive paste layers are provided on the base fabric layer tend to be lower than that in the example 1 in which the one linear electroconductive paste layer is provided on the base fabric layer.

In addition, the following is indicated. The electric resistance value in the example 4 in which the electroconductive paste layer is coated on the entirety of the second base fabric layer surface is a value decreased from that in the comparative example 1. Such a decrease is particularly remarkable as compared with that in the examples 1 to 3 in which the electroconductive paste layer is provided on a part of the second base fabric layer surface. This demonstrates the advantageous effect of the electroconductive rubberized fabric having the three-layer structure that includes the electroconductive paste layer on at least a part thereof. It is also demonstrated that the electroconductive rubberized fabric including the electroconductive paste layer on the entire surface has the particularly remarkable advantageous effect in suppressing an increase in an electric resistance value.

Similarly, the advantageous effect achieved by providing the electroconductive paste layer on the entire surface is recognized also when the electroconductive silicone rubber is used as the electroconductive rubber. In addition, the following is confirmed. The electroconductive rubberized fabric of the example 5 in which the electroconductive paste layer is provided on the entirety of the second base fabric layer surface achieves a more remarkable advantageous effect such as an effect that an electric resistance value of 70 kΩ to 80 kΩ changes at a rate equal to or smaller than approximately 1/1000, thus becoming 50 Ω to 70 Ω, as compared with the comparative example 2.

As described above, it is confirmed that the electroconductive rubberized fabric according to the present example outstandingly suppresses an increase in an electric resistance value by being further provided with the electroconductive paste layer as a third layer on the second base fabric layer surface of the base fabric layer. Thus, it is indicated that the electroconductive rubberized fabric according to the present example can be favorably used as a bioelectrode for accurately detecting a weak biosignal.

### INDUSTRIAL APPLICABILITY

The electroconductive rubberized fabric according to the present invention can be favorably used as a bioelectrode for detecting a biosignal, in any of various electronic devices whose examples include medical devices such as an electroencephalograph, wearable information devices such as an activity amount meter, and on-vehicle devices.

### Reference Signs List

1, 5: Electroconductive rubberized fabric
2: Base fabric layer
2a: Second base fabric layer surface
2b: First base fabric layer surface
3: Electroconductive rubber layer
4, 9: Electroconductive paste layer
6: Base fabric
7: Electroconductive rubber
8: Electroconductive paste material
10a: First terminal
10b: Second terminal
11: Aluminum foil
11a: Distal end of aluminum foil
12: Weight
13a, 13b: Wiring
14a, 14b: Electroconductive member
L1: Sample close-contact section
L2: Terminal-to-terminal distance
S: Sample
T: Tester

## Claims

1. An electroconductive rubberized fabric (1, 5) comprising:
a base fabric layer (2) formed of an insulating base fabric (6) and including a first base fabric layer surface (2b) and a second base fabric layer surface (2a);
an electroconductive rubber layer (3) formed by superimposing electroconductive rubber (7) on the first base fabric layer surface (2b); and
an electroconductive paste layer (4, 9) formed by coating the second base fabric layer surface (2a) with an electroconductive paste material (8) having an electric resistance value lower than that of the electroconductive rubber (7);
**characterized in that**
the electroconductive paste layer (4, 9) covers only a part of the second base fabric layer surface (2a).

2. The electroconductive rubberized fabric (1, 5) according to claim 1,
wherein the electroconductive rubber layer (3) contacts against entirety of the first base fabric layer surface (2b).

3. The electroconductive rubberized fabric (1, 5) according to claim 1,
wherein the second base fabric layer surface (2a) has an elongated shape, and
the electroconductive paste layer (4) extends in an elongated direction of the second base fabric layer surface (2a).

4. The electroconductive rubberized fabric (1, 5) according to any one of claims 1 to 3,
wherein the electroconductive paste layer (4, 9) has a layer thickness equal to or smaller than 1/10 of a total layer thickness of the base fabric layer (2) and the electroconductive rubber layer (3).

5. The electroconductive rubberized fabric (1, 5) according to any one of claims 1 to 4,
wherein the electroconductive rubber (7) is stuck to the base fabric (6) by calender forming such that the base fabric layer (2) and the electroconductive rubber layer (3) are formed integrally with each other.

6. The electroconductive rubberized fabric (1, 5) according to any one of claims 1 to 5,
wherein the base fabric (6) has a porous structure including a plurality of voids inside, and
at least parts of the electroconductive rubber (7) and the electroconductive paste material are mixed with each other in the voids.

7. The electroconductive rubberized fabric (1, 5) according to claim 6,
wherein the base fabric (6) is formed of a nonwoven fabric that is made of an aramid fiber.

8. The electroconductive rubberized fabric (1, 5) according to any one of claims 1 to 7,
wherein the electroconductive rubber (7) is silicone rubber or urethane rubber.

## Patentansprüche

1. Elektrisch leitfähiges gummiertes Gewebe (1, 5), das aufweist:
eine Grundgewebeschicht (2), die aus einem isolierenden Grundgewebe (6) ausgebildet ist und eine erste Grundgewebeschichtoberfläche (2b) und eine zweite Grundgewebeschichtoberfläche (2a) aufweist;
eine elektrisch leitfähige Gummischicht (3), die durch Aufbringen von elektrisch leitfähigem Gummi (7) auf die erste Grundgewebeschichtoberfläche (2b) ausgebildet ist; und
eine elektrisch leitfähige Pastenschicht (4, 9), die durch Beschichten der zweiten Grundgewebeschichtoberfläche (2a) mit einem elektrisch leitfähigen Pastenmaterial (8) ausgebildet ist, das einen elektrischen Widerstandswert aufweist, der niedriger ist als der des elektrisch leitfähigen Gummis (7);
**dadurch gekennzeichnet, dass**
die elektrisch leitfähige Pastenschicht (4, 9) nur einen Teil der zweiten Grundgewebeschichtoberfläche (2a) bedeckt.

2. Elektrisch leitfähiges gummiertes Gewebe (1, 5) nach Anspruch 1,
wobei die elektrisch leitfähige Gummischicht (3) mit der gesamten ersten Grundgewebeschichtoberfläche (2b) in Kontakt steht.

3. Elektrisch leitfähiges gummiertes Gewebe (1, 5) nach Anspruch 1,
wobei die zweite Grundgewebeschichtoberfläche (2a) eine längliche Form aufweist und
sich die elektrisch leitfähige Pastenschicht (4) in einer Längsrichtung der zweiten Grundgewebeschichtoberfläche (2a) erstreckt.

4. Elektrisch leitfähiges gummiertes Gewebe (1, 5) nach einem der Ansprüche 1 bis 3,
wobei die elektrisch leitfähige Pastenschicht (4, 9) eine Schichtdicke aufweist,
die gleich oder kleiner als 1/10 einer Gesamtschichtdicke der Grundgewebeschicht (2) und der elektrisch leitfähigen Gummischicht (3) ist.

5. Elektrisch leitfähiges gummiertes Gewebe (1, 5) nach einem der Ansprüche 1 bis 4,
wobei der elektrisch leitfähige Gummi (7) durch Kalanderbildung so an dem Grundgewebe (6) haftet, dass die Grundgewebeschicht (2) und die elektrisch leitfähige Gummischicht (3) integral miteinander ausgebildet sind.

6. Elektrisch leitfähiges gummiertes Gewebe (1, 5) nach einem der Ansprüche 1 bis 5,
wobei das Grundgewebe (6) eine poröse Struktur aufweist, die mehrere Hohlräume im Inneren aufweist, und
mindestens Teile des elektrisch leitfähigen Gummis (7) und des elektrisch leitfähigen Pastenmaterials in den Hohlräumen miteinander vermischt sind.

7. Elektrisch leitfähiges gummiertes Gewebe (1, 5) nach Anspruch 6,
wobei das Grundgewebe (6) aus einem Vliesstoff ausgebildet ist, der aus einer Aramidfaser besteht.

8. Elektrisch leitfähiges gummiertes Gewebe (1, 5) nach einem der Ansprüche 1 bis 7,
wobei der elektrisch leitfähige Gummi (7) Silikongummi oder Urethangummi ist.

## Revendications

1. Tissu caoutchouté électroconducteur (1, 5), comprenant :
une couche de tissu de base (2) constituée d'un tissu de base isolant (6) et comprenant une première surface (2b) de couche de tissu de base et une deuxième surface (2a) de couche de tissu de base ;
une couche de caoutchouc électroconducteur (3) formée par stratification de caoutchouc électroconducteur (7) sur la première surface (2b) de couche de tissu de base ; et
une couche de pâte électroconductrice (4, 9) formée par revêtement de la deuxième surface (2a) de couche de tissu de base avec un matériau de pâte électroconductrice (8) ayant une valeur de résistance électrique inférieure à celle du caoutchouc électroconducteur (7) ;
**caractérisé en ce que**
la couche de pâte électroconductrice (4, 9) ne recouvre qu'une partie de la deuxième surface (2a) de couche de tissu de base.

2. Tissu caoutchouté électroconducteur (1, 5) selon la revendication 1,
où la couche de caoutchouc électroconducteur (3) est en contact avec la totalité de la première surface (2b) de couche de tissu de base.

3. Tissu caoutchouté électroconducteur (1, 5) selon la revendication 1,
où la deuxième surface (2a) de couche de tissu de base présente une forme allongée, et
où la couche de pâte électroconductrice (4) s'étend dans la direction d'allongement de la deuxième surface (2a) de couche de tissu de base.

4. Tissu caoutchouté électroconducteur (1, 5) selon l'une des revendications 1 à 3,
où la couche de pâte électroconductrice (4, 9) a une épaisseur de couche égale ou inférieure à 1/10^{e} de l'épaisseur totale de la couche de tissu de base (2) et de la couche de caoutchouc électroconductrice (3).

5. Tissu caoutchouté électroconducteur (1, 5) selon l'une des revendications 1 à **4,**
où le caoutchouc électroconducteur (7) est collé au tissu de base (6) par calandrage, de sorte que la couche de tissu de base (2) et la couche de caoutchouc électroconducteur (3) sont formées d'un seul tenant.

6. Tissu caoutchouté électroconducteur (1, 5) selon l'une des revendications 1 à 5,
où le tissu de base (6) a une structure poreuse comprenant une pluralité de cavités intérieures, et
au moins des parties du caoutchouc électroconducteur (7) et du matériau de pâte électroconducteur sont mélangées entre elles dans les cavités.

7. Tissu caoutchouté électroconducteur (1, 5) selon la revendication 6,
où le tissu de base (6) est constitué d'un tissu non tissé en fibre d'aramide.

8. Tissu caoutchouté électroconducteur (1, 5) selon l'une des revendications 1 à 7,
où le caoutchouc électroconducteur (7) est un caoutchouc silicone ou un caoutchouc uréthane.
